# EUROPEAN PATENT APPLICATION

(11) **EP 2 889 030 A1**
(43) Date of publication of application: **01.07.2015**
(21) Application number: 13199748.8
(22) Date of filing: 30.12.2013
(51) Int. Cl.: A61K 9/70

(54) **Controlling the erosion rates of mucoadhesive devices that deliver actives and other compounds and providing increased and variable therapeutic blood levels**

(71) Applicant: Uluru Inc., Addison, TX 75001 (US)
(72) Inventor: Moro, Daniel George, Frisco, TX Texas 75034 (US)
(74) Representative: Gates, Marie Christina Esther

(57) **Abstract**

The present invention relates to the delivery of pharmaceuticals and other compounds to any wet tissue surface with a preferred application on mucosal surfaces. The device consists of a layered film comprising an adhesive layer, an erodible backing layer and an optional outer layer to improve mouth feel. Upon application, the device adheres to a wet tissue surface, and based on the composition of the erodible backing layer which is laminated to the adhesive layer, the device erodes uniformly and at a constant rate from the outside in. Due to this constant and unexpected uniform erosion characteristic, any compound present in the adhesive layer will be delivered to the treatment site at a rate dependent only on the erosion rate of the device. A pseudo zero order delivery rate is attained when an active is delivered on the oral mucosa and this rate can be regulated simply by changing the composition and thickness of the erodible backing layer. This backing layer is composed of both water soluble and water insoluble polymers, and by careful manipulation of the amount of each, the erosion rates can be regulated and subsequent drug delivery rates can be reproducibly altered. In addition, by keeping the device attached to the mucosa for extended and controlled periods of time, the active will pass through the intercellular pathway and provide more rapid and increased therapeutic blood levels. By changing only the backing layer of the device, peak blood levels can be altered.

## Description

### Field of the Invention

This invention relates to the cosmetic or therapeutic treatment of any wet tissue surface and more specifically to an adherent, erodible film that provides one or more active agents, pharmaceutical compounds, nutraceutical compounds, cosmeceutical compounds, vitamins, minerals, botanical extracts, flavors, fragrances or other substances to a wet tissue surface.

### BACKGROUND OF THE INVENTION

The localized treatment of body tissues, diseases and wounds requires that a particular pharmaceutical be administered and maintained at the treatment site for a therapeutically effective period of time. The topical treatment of wet, mucosal surfaces has been problematic, since natural bodily fluids can rapidly wash away a topically applied active compound before the appropriate therapeutic action to the underlying surface can occur. In the mouth, saliva, the natural replacement of the mucosal tissue, and the actions of eating, speaking and drinking are just some of the problems that have limited the usefulness of pharmaceutical carrier devices. Despite these issues, the delivery of actives through the oral mucosa provides many benefits, including attaining rapid therapeutic blood levels, potentially lower amount of drug to provide therapeutic efficacy, convenient application and mandating user compliance. Therefore, this technique represents a viable treatment modality for specific disorders and diseases.

Gels, pastes, tablets and films have been developed as bioadhesive carriers and are well known in the art. These types of products, however, do not exhibit all of the major characteristics required for an efficient and commercially acceptable pharmaceutical delivery device for mucosal treatment. The important characteristics include, water erodibility, ease of handling and application to the treatment site, comfort with minimal foreign body sensation, rapid adhesion, prolonged residence time for the protection of the treatment site and/or the delivery of a pharmaceutical or other active compound, and ease of removal from the underlying mucosal surface by natural erosion or dissolution of the delivery device at the treatment site.

Bioadhesive gels used especially in the oral mucosal cavity are known in the art. For example, U.S. Pat. No.5,192,802 describes a bioadhesive teething gel composed of a mixture of sodium carboxymethyl cellulose and xanthan gum. This gel composition may have potential use in the treatment of canker sores, fever blisters and hemorrhoids. However, these types of gel systems have limited residence times, since bodily fluids such as saliva will quickly wash gels away from the treatment site. Other bioadhesive gels described in U.S.Pat Nos. 5,314,915 ,5,298,258 and 5,642,749 use aqueous or oily medium and different types of bioadhesive and gelling materials, but still suffer from the inherent limitation of all gel products. Another type of bioadhesive products known in the art is denture adhesive pastes. These products, however, were developed primarily for their adhesive properties only, and not to protect tissue or deliver pharmaceuticals to the underlying mucosal surface. However, active compounds such as local anesthetics may be formulated with the paste for the relief of sore gums. Denture adhesive pastes are described in U.S. Pat. Nos. 4,894,232 and 4,551,721. In the'721 patent, the combination of sodium carboxymethyl cellulose and polyethylene oxide in polyethylene glycol is used to provide a bioadhesive composition. Mucoadhesive pastes have also been used as protective films and drug delivery systems. Orabase.RTM.-B, a commercialized paste product that has both film forming and adhesive properties, is used for the relief of mouth sores. This product does provide numbing of the treatment site, but the residence time is small due to the quick dissolution by saliva. In addition, the numbing affect is not localized as the product gets distributed throughout the mouth via the tongue and movement of saliva. This product contains guar gum, sodium carboxymethyl cellulose, tragacanth gum and pectin.

Bioadhesive tablets are described in U.S. Pat. No. 4,915,948. A xanthan gum or a pectin in combination with an adhesion enhancing material such as polyol is the water-soluble bioadhesive used in this device. Although the residence time is greatly enhanced, these tablets are not user friendly, especially when used in the oral cavity, due to their unpleasant feeling, solidity, bulkiness and slow dissolution time. Also, solid devices cannot readily adhere to curved surfaces, especially crevices within the oral cavity. Bioadhesive tablets described in U.S. Pat. Nos. 4,226,848; 4,292,299, and 4,250,163 are single or bilayer devices having an average thickness of 0.2 to 2.5 mm. These devices are less bulky, but have limited residence times. They are composed of a non-adhesive material such as cellulose ether, a bioadhesive ingredient such as polyacrylic acid, sodium carboxymethyl cellulose, or polyvinylpyrrolidone, and a binder for tableting purposes. The cellulose derivatives used in these devices mayor may not be water-soluble. The bilayer devices described in the '299 patent contain methyl cellulose, hydroxypropyl cellulose and hydroxypropylmethyl cellulose. Bandages and bioadhesive laminated films are also known in the art. The films as described in U.S. Pat. Nos. 3,996,934 and 4,286,592 are thinner, more flexible and therefore elicit a decreased foreign body sensation. The laminated films are usually composed of an adhesive layer, a reservoir layer and a backing layer and are designed to deliver drugs through the skin or mucosa. These films are typically not water soluble, thus they are not dissolved or washed away by bodily fluids and must be removed after the prescribed treatment time.

Film delivery systems for use on mucosal surfaces are also known in the art. These types of systems, which are water-insoluble and usually in the form of a laminated, extruded or composite film, are described in U.S. Pat. Nos. 4,517,173; 4,572,832; 4,713,243; 4,900,554 and 5,137,729. The '173 patent relates to a membrane-adhering film composed of at least three layers, including a layer containing a pharmaceutical, a layer with limited water solubility, and an intermediate layer. The pharmaceutical layer contains a drug and a cellulose derivative selected from hydroxypropyl cellulose, methyl cellulose, and hydroxypropylmethyl cellulose. The layer having limited water solubility consists of a combination of one or more cellulose derivatives and a hydrophobic fatty acid, and the intermediate layer is made of cellulose derivatives. The '832 patent describes a.backslash.soft film for buccal delivery. The film is composed of a water soluble protein, a polyol, and a polyhydric alcohol such as cellulose and polysaccharides and coloring and flavoring agents. The '243 patent relates to a single or multi-layered bioadhesive thin film made from 40-95% water soluble hydroxypropyl cellulose, 5-0% water-insoluble ethylene oxide, 0-10% waterinsoluble ethyl cellulose, propyl cellulose, polyethylene or polypropylene and a medicament. These films are three-layered laminates and are composed of a bioadhesive layer, a reservoir layer, and a non water-soluble outer protective layer. The '729 patent teaches a soft, adhesive film for use on oral mucosa. The film is comprised of a mixture of vinyl acetate non water-soluble homopolymer, an acrylic acid polymer, a cellulose derivative and a systemic drug.

In the '554 patent, the device is designed for use in the oral cavity and is composed of an adhesive layer including a mixture of an acrylic acid polymer, a water-insoluble cellulose derivative, a water-insoluble or sparingly soluble backing layer, and a pharmaceutical. The adhesive layer contains the active ingredient and upon application to the treatment site, the drug is delivered to the underlying mucosal surface. This patent also teaches that all three of the aforementioned components are required to attain an appropriate adhesive device suitable for mucosal treatment and drug delivery.

Finally, bioerodable films for the delivery of pharmaceutical compounds are also known in the art. U.S. Pat. Nos. 5,800,832 and 6,159,498 describe a bioerodable, water soluble pharmaceutical device to treat mucosal surfaces. These bilayer devices are composed of an adhesive layer and a non-adhesive backing layer, and the pharmaceutical may be contained in either or both layers.

The composition of the adhesive layer comprises polyacrylic acid, sodium carboxymethyl cellulose, and polyvinyl pyrrolidone, alone or in combination thereof. In addition to these mucoadhesive polymers, film forming polymers such as hydroxyethyl cellulose and hydroxypropyl cellulose are present. This layer can also contain a pharmaceutical compound. The backing layer of these devices comprises only film-forming polymers, such as hydroxyethyl cellulose, hydroxypropyl cellulose and hydroxypropylmethyl cellulose. These polymers are known to exhibit low bioadhesion and are approved for use in a variety of pharmaceutical applications. The residence time is claimed to be regulated by only variations of the backing layer. To increase the residence time, the components of the backing layer can be crosslinked with glyoxal solution, rendering the polymers less water soluble, and therefore, slower to dissolve, while being exposed to bodily fluids like saliva. A second approach is to change the composition of the backing layer by using a mixture of different and higher molecular weight polymers from the same family of hydroxyethyl and hydroxypropyl celluloses. These alterations to the backing layer are easy to accomplish. However, they do not provide a consistent, controllable and reproducible residence time for the final device. In addition, in order to produce this device, it is required to cast the mucoadhesive layer and/or backing layer onto a hard and non-porous surface. Then, each layer is dried yielding a laminated film. The casting surface therefore becomes an integral part of the device or must be carefully removed from the laminated film prior to cutting to the desired shape and subsequent packaging. The associated manufacturing processes to produce such a device are complicated and therefore are not commercially viable or cost effective.

### SUMMARY OF THE INVENTION

The present invention relates to a novel, cost-effective, commercially viable, water-erodible, pharmaceutical carrier device. The device is applied to wet tissue surfaces, especially mucosal surfaces, and provides protection of the application site while delivering pharmaceuticals or other compounds to treat specific diseases, disorders or for cosmetic purposes. The device causes minimum discomfort, is easy to use and provides an effective residence time that can be tailored to deliver therapeutics over different time intervals. In addition to these attributes which describe the device that is disclosed in our related US Patent 6585997, it has been discovered using the basic composition of this mucoadhesive device and the novel erodible backing layer to control residence time, that, unexpectedly, active compounds are delivered to the oral mucosa at a rate proportional to the erosion rate of the device. This is a result of the adhered device eroding from the outside inward, as the edges erode away still leaving the remaining device attached until complete erosion takes place. Any active compound formulated in the adhesive layer that is attached to the oral mucosa will leave the device at a pseudo zero-order release rate. This result has never been disclosed in the literature, as it would be expected that the erosion rate be proportional to the radius of the device. In addition, as expected, peak blood levels containing the respective pharmaceutical can be increased using mucosal delivery, but it has also been discovered that peak blood levels can be altered by changing the composition of the attached backing layer which controls the erosion rate of the device.

The devices described in US Patent 6,585,997 are very effective with respect to delivering actives by controlling the backing layer thickness and composition. However, there are some inherent manufacturing limitations that have been overcome with the current invention. First, the precast film of hydroxypropylmethyl cellulose was used to produce many of the examples outlined in the referenced patent. This film is important to the overall characteristics of the device, since it adds flexibility and overall strength to the final product. It also helps in the uniform erosion rate of the device in vivo. However, since this film is extremely thin and wrinkles easily during scale-up production in the lamination process, inconsistent quality of the final multilayered product result. Also, the thickness variability throughout this precast film is extensive, and this leads to significant variability when subsequent coatings are produced on top of this film. Each additional coating has inherent thickness variability due to the intrinsic variability of the precast film beneath it. It is virtually impossible without losing a significant amount of product to screen out these regions of inconsistent thicknesses. In addition, the content uniformity of the adhesive layer that contains an active becomes variable throughout the laminated film if it is applied on top of the premade film. These issues necessitated an improvement in the manufacturing process. We have discovered that by replacing this premade water soluble film with a comparable film formed in situ from a viscous solution of the same polymeric composition, all of the thickness variability is eliminated. What has essentially been accomplished is a four layered film as described in the issued patent with intrinsic thickness variability is now a three layered film of consistent thickness throughout. Not only is the quality of the product significantly improved, but the cost to produce it is more economical, since only three coating passes instead of four are required and the yield is dramatically improved. The actual process and differences between the two manufacturing techniques are outlined in the examples following this disclosure.

In one embodiment, the device comprises a mucoadhesive multi-layered film that is water-soluble and bioerodible. In another embodiment, the pharmaceutical delivery device comprises a multi-layered film having an adhesive layer and a coated backing layer containing a pharmaceutical or other active compound in either or both layers. The film may be cut or fabricated into any desired shape, such as a disc, square, oval, parallelepiped, etc., that provides convenience for use in application and/or treatment. The adhesive layer of the device is water soluble and the backing layer is bioerodible. The adhesive layer comprises a filmforming polymer such as hydroxyethyl cellulose, hydroxypropyl cellulose, hydroxypropylmethyl cellulose, or hydroxyethylmethyl cellulose, alone or in combination, and a bioadhesive polymer such as polyacrylic acid, polyvinyl pyrrolidone, sodium carboxymethyl cellulose, methyl cellulose, chitosan alone or in combination. The non-adhesive backing layer is a water soluble film, either precast or formed in situ, alone or in combination with other layers. The water soluble film is comprised of hydroxyethyl cellulose, hydroxypropyl cellulose, hydroxyethylmethyl cellulose, hydroxypropylmethyl cellulose, polyvinyl alcohol, polyethylene glycol, polyethylene oxide, ethylene oxide-propylene oxide copolymers, or other water soluble film-forming polymer, alone or in combination thereof. This film may also include plasticizers or other excipients required to enhance the film forming properties of the polymer. The non-adhesive backing layer is further modified to render it water erodible instead of water soluble. For definition purpose, water erodible means a material or substance that does not dissolve in water or bodily fluids in total, however will disintegrate and completely break apart upon exposure to water or bodily fluids. This is accomplished by coating it with a mixture of both hydrophilic and hydrophobic polymers. As this coating layer is exposed to saliva, the water soluble (hydrophilic) polymer(s) comprising this layer dissolve, leaving behind a weak film that will disintegrate over time due to the reduced mechanical strength. The hydrophobic polymers are selected from a group of Eudragit.RTM. and/or ethyl cellulose and methyl cellulose polymers that are approved by the FDA for use in pharmaceutical applications. Other hydrophobic polymers known to those skilled in the art may also be used. The type and amount of hydrophobic polymer used will provide a wide and controlled range of Residence Times and subsequent erosion rates for the layered disk device. The water soluble polymers that comprise this coating layer consist of hydroxyethylmethyl cellulose, hydroxyethyl cellulose, polyvinyl pyrrolidone, polyvinyl alcohol and other know pharmaceutically acceptable water soluble polymers. An optional water soluble layer can be coated on top of this water erodible coating layer to improve mouth feel.

An important aspect of the present invention concerns a mucoadhesive erodible multilayered device placed in contact with a mucosal surface and second water erodible non-adhesive backing layer that controls residence time and erosion rate of the device. Residence time, the time for which device in placed on the target mucosal surface will remain substantially intact. The first layer comprises at least one water soluble film forming element in combination with at least one mucoadhesive polymer. The second water erodible non adhesive backing layer comprises a water soluble film, either precast or formed in situ containing at least one of hydroxypropyl methylcellulose, hydroxyethyl cellulose, hydroxypropyl cellulose, polyvinyl alcohol, polyethylene glycol, polyethylene oxide, and ethylene oxide-propylene oxide co-polymer. This layer is coated with at least one hydrophobic polymer alone or in combination with at least one hydrophilic polymer, such that the layer is bioerodible. An optional water soluble layer can be added as the final layer to improve mouth feel.

This device may be used by itself for cosmetic and/or non-Rx applications or may be used with an incorporated pharmaceutical agent. This device may be used for the protection of a mucosal site and/or the administration of a pharmaceutical agent locally, regionally or systemically. In a preferred embodiment, the first water-soluble adhesive layer comprises at least one watersoluble film-forming polymer selected from the group consisting of hydroxyethyl cellulose, hydroxypropyl cellulose, hydroxypropyl methylcellulose, and hydroxyethyl methylcellulose, in combination with at least one mucoadhesive polymer selected from the group consisting of polyacrylic acid, polyvinyl pyrrolidone, methyl cellulose, chitosan , sodium carboxymethyl cellulose, alone or in combination. This second layer preferably comprises a film produced in situ of hydroxypropyl methylcellulose in combination with a coating consisting of at least one hydrophobic polymer selected from the family of Eudragit polymers, ethyl cellulose and methylcellulose alone or in combination with at least one hydrophilic polymer selected from the group consisting of polyvinyl pyrrolidone, hydroxypropylmethylcellulose, hydroxyethylmethylcellulose, hydroxypropylcellulose and polyvinylalcohol.

In certain preferred embodiments the mucoadhesive bioerodible multilayered device of the present invention has a second water erodible non-adhesive backing layer that comprises a film of hydroxypropyl methylcullulose and a coating of a hydrophobic and hydrophilic polymer mixture at a ratio of 5:1 to 1:1. A more preferred ratio is 3:0 to 1:1.

In an important embodiment, the non-adhesive backing layer of the device of the present invention comprises a film of hydroxypropyl methylcellulose with a coating mixture of hydrophobic and hydrophilic polymers at a ratio of 5 to:1 to 1:1. This coating contains at least one of propylene glycol, polyethylene glycol or glycerine as a plasticizer to improve flexibility. A preferred non-adhesive backing layer of the device of the present invention comprises a film of hydroxypropyl methylcellulose, either precast or formed in situ and a coating mixture of hydrophobic and hydrophilic polymers at a ratio of 5:1 to 1:1. A preferred coating mixture may contain at least one of hyaluronic acid and an alpha hydroxyl acid as a humectant to improve softness or feel. A preferred humectant is glycolic acid.

In one particularly preferred embodiment, the mucoadhesive erodible multi layered device of the present invention has a non-adhesive backing layer that comprises a film of hydroxypropyl methylcellulose and a coating mixture of hydrophobic and hydrophilic polymers at a ratio of 5:1 to 1:1. A preferred embodiment of the mucoadhesive layer contains titanium dioxide, zinc oxide or zirconium silicate as an opacifier and a preferred coating mixture contains one or more FD&C Red, Yellow, Green or Blue as a coloring agent to help distinguish the backing layer from the mucoadhesive layer. In one embodiment, the backing layer of the present device comprises a film of hydroxypropyl methylcellulose, a coating comprising a mixture of hydrophobic and hydrophilic polymers at a ratio of 5:1 to 1:1, a plasticizer and a coloring agent whose combined total is less than about 4% by weight of the device.

In a very important embodiment of the present invention, the mucoadhesive, erodible multi-layered device further comprises at least one pharmaceutical agent incorporated within said first or second layer. The pharmaceutical agent is defined wherein any agent that has desirable prophylatic or curative effects. Pharmaceutical agents may be incorporated within the first or second layers of the device of the present invention. These layers may each independently comprise flavoring agent to mask the taste of any pharmaceutical agent or simply to improve patient compliance.

One class of pharmaceutical agents is anti-allergic compounds. Anti-allergic agents may be, e.g., amlexanox, astemizole, azelastinep, emirolast, alopatadine, cromolyn, fenpiprane, repirinast, tranilast, or traxanox.

One pharmaceutical agent that may be a component of the device of the present invention is an anti-inflammatory analgesic agent. Such anti-inflammatory analgesic agents may be, e.g., acetaminophen, methyl salicylate, monoglycol salicylate, aspirin, mefenamic acid, flufenamic acid, indomethacin, diclofenac, aldlofenac, diclofenac sodium, ibuprofen, ketoprofen, naproxen, pranoprofen, fenoprofen, sulindac, fenclofenac, clidanac, flubiprofen, fentiazac, bufexarnac, piroxicam, phenylbutazone, oxyphenbutazone, c1 ofezone, pentazocine, mepirizole, or tiaramide hydrochloride. In some cases the pharmaceutical agent of the present invention may be an antianginal agent. Such antianginal agents may be nifedipine, atenolol, bepridil, carazolol, or epanolol.

Steroidal anti-inflammatory agents may sometimes comprise the pharmaceutical agents of the present invention. Steroidal anti-inflammatory agents may be, e.g., hydrocortisone, predonisolone, dexamethasone, triamcinolone acetonide, fluocinolone acetonide, hydrocortisone acetate, predonisolone acetate, methylpredonisolone, dexamethasone acetate, betamethasone, betamethasone valerate, flutetasone, fluormetholone, or orbeclomethasone dipropionate.

Another usable class of pharmaceutical agents is antihistamines. Such antihistamines may be, e.g., diphenhydramine hydrochloride, chlorpheniramine maleate, isothipendyl hydrochloride, tripelennamine hydrochloride, promethazine hydrochloride, or methdilazine hydrochloride.

The pharmaceutical agent of the present invention may be a local anesthetic, such as, e.g., dibucaine hydrochloride, dibucaine, lidocaine hydrochloride, lidocaine, benzocaine, p-buthylaminobenzoic acid, 2-(di-ethylamino) ethyl ester hydrochloride, procaine hydrochloride, tetracaine, tetracaine hydrochloride, chloroprocaine hydrochloride, oxyprocaine hydrochloride, mepivacaine, cocaine hydrochloride, piperocaine hydrochloride, dyclonine, or dyclonine hydrochloride.

When the preferred pharmaceutical agent is a bactericide or disinfectant, it may, e.g., be thimerosal, phenol, thymol, benzalkonium chloride, chlorhexidine, povidone iodine, cetyl- pyridinium chloride, eugenol, trimethylanmmonium bromide, benzoic acid or sodium benzoate.

When the pharmaceutical agent of the present invention is a vasoconstrictor, it may be, e.g., naphazoline nitrate, tetrahydrozoline hydrochloride, oxymetazoline hydrochloride, phenylephrine hydrochloride, or tramazolinehydrochloride.

When the pharmaceutical agent incorporated in the device of the present invention is a hemostatic agent, preferably is, e.g., thrombin, phytonadione, protamine sulfate, aminocaproic acid, tranexamic acid, carbazochrome, carbaxochrome sodium sulfate, rutin, or hesperidin.

If the pharmaceutical agent in the device of the present invention is a chemotherapeutic agent, it may be, e.g., sulfamine, sulfathiazole, sulfadiazine, homosulfamine, sulfisoxazole, sulfisomidine, sulfamethizole, nitrofurazone, taxanes, platinum compound, topoisomerase 1 inhibitor, or anthracycline.

When the pharmaceutical agent incorporated in the device of the present invention is an antibiotic it may be, for example, penicillin, meticillin,oxacillin, cefalotin, cefalordin, erythromycin, lincomycin, tetracycline, chlortetracycline, oxytetracycline, chloramphenicol, kanamycin, streptomycin, gentamicin, bacitracin, cycloserine, or clindamycin.

The pharmaceutical agent of the present invention when incorporated into a device as described herein may be a keratolytic agent. Such a keratolytic agent may be, e.g., alicylic acid, podophyllum resin, podolifox, or cantharidin.

When the pharmaceutical agent incorporated into the device of the present invention is a cauterizing agent, it may be chloroacetic acid or silver nitrate.

The pharmaceutical agent incorporated in a device of the present invention may also be a hormone. Such a hormone may be, e.g., estrone, estradiol, testosterone, equilin, or human growth hormone.

This pharmaceutical agent that may be incorporated in a device of the present invention may also be a growth hormone inhibitor. Preferred growth hormone inhibitors include octreotide or somatostatin.

If the incorporated pharmaceutical agent is an analgesic narcotic it may be, for example, fentanyl, buprenorphine, codeine sulfate, levophanol, or morphine hydrochloride.

If this pharmaceutical agent is an antiviral agent, it may be, for example, protease inhibitor, thymidine kinase inhibitor, sugar or glycoprotein synthesis inhibitor, structural protein synthesis inhibitor, attachment and adsorption inhibitor, or nucleoside analogue.

Such a nucleoside analogue may be acyclovir, penciclovir, valacyclovir, or ganciclovir.

In the most preferred embodiments in the present invention, when pharmaceutical agents are added, they are between 0.001 percent and about 30.00 percent by weight of the device. More preferably, the pharmaceutical agent is between about 0.005 and about 20 percent by weight of the device.

In applications that do not use a pharmaceutical, the flavor, nutraceutical, or other compound is present between 0.01 and about forty per cent, and in the most preferred embodiment, these compounds are between 0.1 and thirty percent.

### DETAILED DESCRIPTION OF THE EMBODIMENTS

### Definitions

As used in the specification and claims, the singular form "a", "an" and "the" include plural references unless the context clearly dictates otherwise. For example, the term "a layer" includes more than one layer.

As used herein, the term "comprising" is intended to mean that the compositions and methods include the recited elements, but not excluding others. "Consisting essentially of" when used to define compositions and methods, shall mean excluding other elements of any essential significance to the combination. "Consisting of" shall mean excluding more than trace elements of other ingredients and substantial method steps for using the devices of this invention. Embodiments defined by each of these transition terms are within the scope of this invention.

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about". It also is to be understood, although not always explicitly stated, that the reagents described herein are merely exemplary and that equivalents of such are known in the art.

As used herein, a "surfactant" has the meaning generally understood by those skilled in the chemical art. That is, a surfactant is a soluble compound, which may be anionic, cationic, zwitterionic, amphoteric or neutral in charge, and which reduces the surface tension of the liquid in which it is dissolved or that reduces interfacial tension between two liquids or a liquid and a solid.

As used herein, the term "monomer" has the meaning understood by those skilled in the chemical art. That is, a monomer is a small chemical compound that is capable of forming a macromolecule of repeating units of itself, i.e., a polymer. Two or more different monomers may react to form a polymer in which each of the monomers is repeated numerous times, the polymer being referred to as a copolymer to reflect the fact that it is made up of more than one monomer.

As used herein, an "active agent" refers to any substance that is other than a carrier or excipient. Examples of active agents include, for example, biomedical agents; biologically active substances such as pharmaceutical agents, genes, proteins, growth factors, monoclonal antibodies, fragmented antibodies, antigens, polypeptides, DNA, RNA and ribozymes; agrichemical agents (herbicides, fungicides, insecticides, plant growth hormones, etc.); radiopaque substances; radioactive substances, pigments; dyes; metals; semiconductors; do pants; chemical intermediates; acids; and, bases. An additional example is a "pharmaceutical agent" which refers to both small molecule and to macromolecular compounds used as drugs. Among the former are, without limitation, antibiotics, chemotherapeutics (in particular platinum compounds and taxol and its derivatives), analgesics, antidepressants, anti-allergenics, antiarryhthics, anti-inflammatory compounds, CNS stimulants, sedatives, anticholinergics, anti-arteriosclerotics, and the like. Macromolecular compounds include, without limitation, monoclonal antibodies (mAbs), monoclonal antibody fragments (Fabs), proteins, peptides, cells, antigens, nucleic acids, enzymes, growth factors and the like. A pharmaceutical agent may be intended for topical or systemic use.

As used herein, a "cross-linking agent" refers to a di-, tri-, or tetra-functional chemical entity that is capable of forming covalent bonds with functional groups on polymeric strands resulting in a three-dimensional structure.

As used herein, the term "ex vivo" refers to any process or procedure being performed outside of a living organism, for instance, without limitation, in a Petri dish, in soil, in surface water, in a liquid organic medium and the like.

As used herein, the term "in vivo" refers to any process or procedure performed within a living organism, which may be a plant or an animal, in particular, in a human being.

As used herein, the term "in situ" refers to any process or procedure performed during the manufacture of the device, in which a layer is formed directly in place from a polymeric solution.

As used herein, the term "polymer" shall mean a high-molecular weight substance made up by the repetition of some simpler unit, for example, the monomer.

As used herein, the term "hydrophobic polymer" shall mean any polymer that has little or no affinity for water, does not imbibe water and is not soluble or dispersible in water without the aid of a solubilizing agent.

As used herein, the term "water-soluble polymer" shall mean any polymer that has affinity for water, is soluble or uniformly dispersible in water without the aid of a solubilization agent.

As used herein, the term "water-erodible coating layer" shall mean a distinct layer composed of hydrophobic and water-soluble polymers in a specific ratio such that upon exposure to physiological body fluid, e.g., saliva, the water-soluble polymer dissolves from the layer leaving behind a weakened layer that eventually erodes away due to mechanical action.

"Residence Time" is the time the multi-layered device remains on a moist surface of a body tissue until complete erosion.

"Erosion Rate" is the rate at which device erodes as expressed per per cent per unit time

A "plasticizer" is additive that softens and imparts flexibility to a rigid material, typically a polymer, by swelling the amorphous region and lowering the cohesion between the polymer chains.

A "humectant" is a substance that promotes the retention of water.

The term "a coloring agent" shall mean a substance that imparts color or changes the tint or shade of an existing color of a material.

An "opacifying agent" is a substance that reduces the clarity of a material.

A "taste-masking agent" is a substance that is added to a formulation to mask or reduce the unpleasant taste of an active ingredient by promoting a powerful palatable oral sensory-directed reaction.

Mechanically bonded" as used herein shall mean a physical bond accomplished by such means of promoting interlocking macromolecular filaments that are not chemically (ionically or covalently) bonded, through hydrophobic and/or hydrophilic interactions at the surface.

A "nutraceutical" is a substance that improves a product's heath attributes for the expressed purpose of treatment or prevention of disease.

A "cosmeceutical" is a cosmetic that contains biologically active ingredients that claims to have medical benefits.

As used herein, the term "Tₘₐₓ" refers to the length of time required to reach maximum concentration in blood for a given active compound as detected.

As used herein, the term "Cₘₐₓ"refers to the maximum concentration of a given active compound that is attained in blood.

### DETAILED DESCRIPTION OF THE INVENTION

In the present invention, a unique bioerodible miltilayered device that adheres to mucosal surfaces is provided. The erosion rate device can be precisely regulated from about 15 minutes to two hours, based on the composition and thickness of the backing layer. For clarification purposes, the backing layer of these devices consist of a water soluble film, either precast or formed in situ on an adhesive layer, and then coated with a mixture of water soluble and water insoluble polymers rendering the combined backing layer water erodible. An optional water soluble layer can also be attached to the backing layer to improve mouth feel. The present invention is most applicable to the treatment of body tissues, diseases, or wounds that may have moist surfaces and that are susceptible to bodily fluids, such as the mouth, vagina, anus, or other types of wet tissue surfaces. Pharmaceuticals or other active compounds can be incorporated in the device, and upon application and adherence to the specific mucosal site, protection of the underlying tissue results.

Concomitantly, pharmaceuticals are delivered to the treatment site, the surrounding tissues and other bodily fluids for a prolonged period of time. Since the device erodes from the outside inward at a constant rate, the active compound is delivered at a pseudo-zero order rate and its rate is proportional to the erosion rate of the device. The device provides an appropriate, controlled residence time for effective drug delivery at the treatment site. The residence time and erosion rate is easily tailored to provide a range from minutes to hours, dependent upon the type of drug used and therapeutic indication. In one embodiment, the pharmaceutical delivery device comprises a layered film disc having a water soluble adhesive layer and a water erodible backing layer, having a pharmaceutical in either or both layers. By delivering pharmaceuticals directly through the intercellular pathway of the oral mucosa, blood levels are attained more rapidly and can be modified by changing the composition of the backing layer which affects erosion of the device.

The present invention offers advantages with respect to increased residence time over bioadhesive gels and pastes known in the art. Paste and gel products such as Orajel, Orabase, and Kanka have short residence times in the order of minutes. This is a result of limited or poor adhesion. Upon application of a gel product to the mucosal surface, the mucoadhesive components do not instantaneously penetrate the lipophilic surface of the mucosa. Instead, these hydrophilic components quickly mix with saliva or other bodily fluids and therefore are removed from the application site resulting in a minimal residence time. A similar mechanism of action can be expected to occur with paste products, however to a slightly lesser extent. This is due to the higher viscosity and greater hydrophobicity of the paste causing a slower erosion process to occur. The film of the present invention provides for immediate adhesion to the mucosal surface due to the combination of mucoadhesive polymers within water-soluble film-forming polymers, and its thin, flexible solid form. A solid device will dissolve or erode more slowly than a gel or paste device due to dissolution kinetics. Bioadhesive tablets known in the art also have serious limitations, primarily due to their bulkiness and rigidity causing an unpleasant sensation and discomfort after application to the oral cavity. These tablets provide effective residence times, but because they are an order of magnitude larger than the device in the present invention, the preferred application site is on the upper gingival or sublingual area. This site is suitable for systemic delivery of an active compound, but may not be satisfactory for localized, unidirectional delivery. The device of the present invention offers both local and systemic delivery with an effective and controlled residence time and minimal discomfort and ease of application as a result of its thinner, more flexible configuration.

Finally, film systems known in the art that are used to deliver pharmaceuticals also have other limitations. These films, unlike the pharmaceutical device of the present invention, are occlusive and water insoluble and are fabricated to be removed after treatment of a mucosal surface. Removal of a non-erodible device may cause some damage to the mucosa, or may damage healing mucosa when the device is used to cover a lesion. The pharmaceutical device of the present invention is designed to be water erodible, and therefore does not require removal. Once applied to a mucosal surface, water absorption softens the device, and over time, the device slowly erodes away delivering a specific pharmaceutical to the treatment site. In one embodiment, the present invention is composed of a multi-layered film having an adhesive layer, a water soluble non-adhesive backing layer either precast or formed in situ, and a hydrophobic/hydrophilic coating layer. The hydrophobic /hydrophilic coating layer renders the water soluble non-adhesive backing layer water erodible instead of water soluble and provides a wide range of predictable and controlled residence times or erosion rates of the device. The adhesive layer is water soluble and the backing layer is water erodible. All components used to manufacture the device are FDA approved materials. The pharmaceutical or other active agent may be included in either layer, but is preferably incorporated in the adhesive layer. This layer is in direct contact with the treatment site and the active compound will be released at a rate related to the erosion of the adhesive layer. The backing layer will control the rate at which the adhesive layer hydrates, therefore control the rate of dissolution of the adhesive layer.

The adhesive layer may comprise at least one film-forming water-soluble polymer, typically selected from a family of cellulose polymers (the "film-forming polymer") and at least one or more polymers known in the art for its bioadhesive property (the "bioadhesive polymer"). The film-forming polymer may comprise hydroxyethyl cellulose, hydroxypropyl cellulose, hydroxypropylmethyl cellulose, hydroxyethylmethyl cellulose, alone or in combination thereof. The molecular weight of the film-forming polymer is in the range of 10.sup.2 to 10.sup.6, and more preferably between 10.sup.3 to 10.sup.5. The film-forming polymer may be crosslinked and/or the adhesive layer plasticized to alter the dissolution characteristics. The bioadhesive polymer contained in the adhesive layer may comprise polyacrylic acid(PAA), which mayor may not be partially crosslinked, sodium carboxymethyl cellulose(NaCMC), polyvinyl pyrrolidone, ,methyl cellulose, chitosan alone or in combination thereof. These bioadhesive polymers are preferred because they exhibit good instantaneous mucoadhesive properties in the dry, film state. In the case of sodium carboxymethyl cellulose, typical average molecular weights range between 50,000 and 700,000 Daltons, and preferably between 60,000 and 500,000 Daltons, with a degree of substitution of 0.7. The substitution range varies between 0.5 and 1.5, and preferably between 0.6 and 0.9. The polyvinyl pyrrolidone can be characterized according to its average molecular weight and comprises between 5,000 and 150,000 Daltons, preferably between 10,000 and 100,000 Daltons. In some instances, the combination of some grades of polyvinyl pyrrolidone with polyacrylic acid may result in precipitation, causing a non-homogeneous adhesive layer to result and a potentially less than optimum mucoadhesive property. Such combinations of polyacrylic acid and polyvinyl pyrrolidone should be avoided. Other mucoadhesive polymers or combination of mucoadhesive polymers known in the art may also be used.

The chemical nature of the bioadhesive polymers used in the present invention, including chain, side groups and crosslinking agents, generates interactions between the mucosal constituents and the polymer or polymers, such as physical entanglement, Van der Waals forces, and hydrogen bonding. Since the mucosal surface differs from one individual to another and changes naturally over time, the use of a combination of at least two bioadhesive polymers and/or the use of a combination of different grades of the same polymer will provide maximum adhesion of the device for a wide range of different mucosal surfaces. However, the use of a single mucoadhesive polymer is effective as well. The ratio of bioadhesive polymer to film-forming polymer in the adhesive layer can be varied and depends upon the type and amount of pharmaceutical or other active ingredient used and other factors. However, the content of combined components in the adhesive layer is between 5 and 95% by weight, preferably between 10 and 80% by weight. In terms of weight percent of the different bioadhesive polymers PAA, NaCMC, and PVP, some examples are detailed later. Preferred combinations include PAA and NaCMC, NaCMC and PVP, or PAA and PVP, and also include the use of different molecular weight grades of the same polymer

The non-adhesive backing layer is a water soluble, film-forming, pharmaceutically acceptable polymer such as hydroxyethyl cellulose, hydroxypropyl cellulose, hydroxyethylmethyl cellulose, hydroxypropylmethyl cellulose, polyvinyl alcohol, polyethylene glycol, polyethylene oxide, ethylene oxide-propylene oxide co-polymers, or other water soluble film-forming polymers, alone or in combination thereof. The non-adhesive backing layer is further modified to render it water erodible instead of water soluble. This is accomplished by coating the backing layer film, which is either a precast film or is formed directly in situ on top of the adhesive layer with a solution composed of both hydrophilic and hydrophobic polymers. The hydrophobic polymers are selected from a group of FDA approved Eudragit polymers, ethyl cellulose and methyl cellulose polymers that are approved for use in other pharmaceutical dosage forms. Other hydrophobic polymers may be used, alone or in combination with other hydrophobic or hydrophilic polymers, provided that the layer derived from these polymers or combination of polymers erodes in a moist environment. The hydrophilic polymer comprising this coating layer is selected from the group consisting of polyvinyl pyrrolidone, hydroxypropylmethylcellulose, hydroxyethylmethylcellulose, hydroxypropylcellulose and polyvinylalcohol. An optional water soluble coating layer can be applied on top of this coating layer to improve mouth feel.

The type of solution used to coat the water soluble backing layer typically is composed of a mixture of a hydrophobic Eudragit polymer or copolymer, or ethyl or methyl cellulose, and a water soluble polymer such as polyvinyl pyrrolidone, hydroxypropyl methyl cellulose, hydroxyethyl cellulose, hydroxypropyl cellulose, polyvinyl alcohol or any other water soluble polymer that can be completely comixed with Eudragit or ethyl cellulose, dissolved in Ethanol or other suitable volatile organic solvent. The ratio of hydrophobic Eudragit or ethyl cellulose to water soluble polymer ranges from 5:1 to 1:1, and more preferably 3:1 to 1:1. The coating solution may also contain a plasticizing agent, such as propylene glycol, polyethylene glycol, or glycerine in a small amount, 0 to 2% by weight, in order to improve the "flexibility" of this layer in the mouth. In addition, humectants such as hyaluronic acid, glycolic acid, and other alpha hydroxyl acids can also be added to improve the "softness" and "mouth feel" of the device. Finally, colors and opacifiers may be added to help distinguish the non-adhesive backing layer from the mucoadhesive layer. Some opacifers include titanium dioxide, zinc oxide, zirconium silicate, etc.

The amount of mixed coating applied to the backing layer, using a suitable doctor blade or lab coater apparatus, ranges between zero and 1.5 mm, most preferably between 0.05 and 0.4 mm. The amount of solids present in the coating solution, the resulting solution viscosity and coating thickness applied determine the amount of coating film to be deposited on the water soluble layer. Increasing the hydrophobic polymer to water soluble polymer ratio will provide a device with a longer residence time, while keeping coating thickness, viscosity, coating solids, polymer composition, and other variables constant. In addition, increasing the coating thickness of the Eugragit and/or ethyl cellulose/water-soluble polymer mixture while keeping all other variables constant will also provide an increased residence time. This will be clearly shown in examples shown later in this disclosure.

The residence time or erosion rate of the device of the present invention is dependent upon the composition of the modified backing layer and the rate of dissolution of the water-soluble polymers used. The residence time is easily controlled, from minutes to hours, by the amount of coating solution applied to the backing layer and the specific composition of the coating solution. The device of the present invention is therefore more versatile than those films, tablets and gels known in the art and can be used with a wider range of pharmaceuticals or other active compounds requiring different therapeutic delivery times. The device of the present invention also typically provides, when desired, a longer residence time than those devices known in the art. This is a result of the selection of the appropriate backing layer formulation, providing a slower rate of erosion of the backing layer and therefore allowing the mucoadhesive layer to remain in contact with the treatment site for a longer period of time before complete erosion. The device of the present invention will maximize the unidirectional delivery of an active compound to the treatment site while minimizing the systemic delivery of the drug that results from surface erosion due to saliva or other bodily fluids. By keeping the device attached to the oral mucosa for extended periods of time, therapeutic blood levels can be attained much more rapidly and these levels are altered by changes in the composition and/or thickness of the coated backing layer. The devices of the present invention are more therapeutically effective for most indications than those devices known in the art, since a sustained level of drug is delivered at a more controlled rate over a longer treatment time. In addition, the present invention describes a therapeutically effective device that can erode at a constant rate, independent of the radius of the device, and thus provide a pseudo zero order drug delivery rate.

The pharmaceutical agent or other active compound of the present invention may comprise a single pharmaceutical or a combination of pharmaceuticals. These active ingredients may be incorporated in the adhesive layer, backing layer or in both. If desired, flavoring agents known in the art may be added to mask the taste of the active compound. Penetration enhancers may also be included in the adhesive layer to help reduce the resistance of the mucosa to drug transport. Typical enhancers known in the art include ethylenediamine tetracetic acid, chitosans, dimethyl sulfoxide, etc. Ingredients to enhance drug solubility and/or stability of the drug may also be added to the layer or layers containing the active ingredient. Examples of stabilizing and solubilizing agents are cyclodextrins.

Pharmaceuticals that may be used, either alone or in combination, include antiallergic compounds, antianginal agents, anti-inflammatory analgesic agents, steroidal anti-inflammatory agents, antihistamines, local anesthetics, bactericides and disinfectants, vasoconstrictors, hemostatics, ace inhibitors, chemotherapeutics, antibiotics, keratolytics, cauterizing agents, hormones, growth hormones and growth hormone inhibitors, analgesic narcotics, and antiviral drugs.

Examples of antiallergic compounds include amlexanox, astemizole, azelastinep, emirolast, alopatadine, cromolyn, fenpiprane, repirinast, tranilast, and traxanox.

Examples of antianginal agents include nifedipine,atenolol, bepridil, carazolol, and epanolol.

Examples of anti-inflammatory analgesic agents include acetaminophen, methyls alicylate, monoglycol salicylate, aspirin, mefenamic acid, flufenamic acid, indomethacin, diclofenac, alclofenac, diclofenac sodium, ibuprofen, ketoprofen, naproxen, pranoprofen, fenoprofen, sulindac, fenclofenac, clidanac, flurbiprofen, fentiazac, bufexamac, piroxicam, phenylbutazone, oxyphenbutazone, clofezone, pentazocine, mepirizole, tiaramide hydrochloride, etc.

Examples of steroidal anti-inflammatory agents include hydrocortisone, predonisolone, dexamethasone, triamcinolone acetonide, fluocinolone acetonide, hydrocortisone acetate, predonisolone acetate, methylpredonisolone, dexamethasone acetate, betamethasone, betamethasone valerate, flumetasone, fluorometholone, beclomethasone diproprionate, etc.

Examples of antihistamines include diphenhydramine hydrochloride, diphenhydramine salicylate, diphenhydramine, chlorpheniramine hydrochloride, chlorpheniramine maleate isothipendyl hydrochloride, tripelennamine hydrochloride, promethazine hydrochloride, methdilazine hydrochloride, etc.

Examples of local anesthetics include dibucaine hydrochloride, dibucaine, lidocaine hydrochloride, lidocaine, benzocaine, p-buthylaminobenzoic acid 2-(diethylamino) ethyl ester hydrochloride, procaine hydrochloride, tetracaine, tetracaine hydrochloride, chloroprocaine hydrochloride, oxyprocaine hydrochloride, mepivacaine, cocaine hydrochloride, piperocaine hydrochloride, dyclonine, dyclonine hydrochloride, etc.

Examples of bactericides and disinfectants include thimerosal, phenol, thymol, benzalkonium chloride, benzethonium chloride, chlorhexidine, povidone iodine, cetylpyridinium chloride, eugenol, trimethylammonium bromide, benzoic acid, sodium benzoate, etc.

Examples of vasoconstrictors include naphazoline nitrate, tetrahydrozoline hydrochloride, oxymetazoline hydrochloride, phenylephrine hydrochloride, tramazoline hydrochloride, etc.

Examples of hemostatics include thrombin, phytonadione, protamine sulfate, aminocaproic acid, tranexamic acid, carbazochrome, carbaxochrome sodium sulfanate, rutin, hesperidin, etc.

Examples of chemotherapeutic drugs include sulfamine, sulfathiazole, sulfadiazine, homosulfamine, sulfisoxazole, sulfisomidine, sulfamethizole, nitro furazone, taxanes, platinum compounds, topoisomerase I inhibitors, and anthrocycline.

Examples of antibiotics include penicillin, meticillin, oxacillin, cefalotin, cefalordin, erythromycin, lincomycin, tetracycline, chlortetracycline, oxytetracycline, metacycline, chloramphenicol, kanamycin, streptomycin, gentamicin, bacitracin, cycloserine, and clindamycin.

Examples of keratolytics include salicylic acid, podophyllum resin, podolifox, and cantharidin. Examples of ace inhibitors include benazepril and benazeprilat.

Examples of cauterizing agents include the chloroacetic acids and silver nitrate.

Examples of hormones include estrone, estradiol, testosterone, equilin, and human growth hormone.

Examples of growth hormone inhibitors are octreotide and somatostatin.

Examples of analgesic narcotics include fentanyl, buprenorphine, codeine sulfate, levorphanol, and morphine hydrochloride.

Examples of antiviral drugs include protease inhibitors, thymidine kinase inhibitors, sugar or glycoprotein synthesis inhibitors, structural protein synthesis inhibitors, attachment and adsorption inhibitors, and nucleoside analogues such as acyclovir, penciclovir, valacyclovir, and ganciclovir.

The amount of active agent or pharmaceutical(s) in the device of the present invention depends upon the therapeutic requirements, although, preferably, the pharmaceutical component comprises 0.001 to 30% by weight of the device, and more preferably between 0.005 and 20% by weight.

Flavoring agents, preservatives, plasticizers, opacifiers, stabilizing and solubilizing compounds, penetration enhancers and coloring agents may also be included in the adhesive layer, backing layer, or both layers of the device. Preferably, these components represent no more than 5% by weight of the final device.

The thickness of the device may vary, depending upon the thickness of each individual layer. Preferably, the thickness of the layered disc of the present invention ranges from 0.05 mm to 1 mm, and more preferably from 0.1 to 0.5 mm. The thickness of each individual layer may vary from 10 to 90% of the overall thickness of the layered device, and preferably varies from 30 to 60%. Therefore, the preferred thickness of each layer may vary from 0.002 mm to 0.9 mm, and more preferably from 0.003 to 0.6 mm.

The pharmaceutical delivery device of the present invention may be prepared by various methods known in the art. In one embodiment, the components are dissolved in the appropriate solvent or combination of solvents to prepare a solution. Solvents for use in the present invention may comprise water, methanol, ethanol, isopropyl alcohol, acetone, methyl ethyl ketone, heptane, or dichloroethane, alone or in combination. The residual solvent content in the dried, multilayered film may act as a plasticizer, an erosion-rate-modifying agent or may provide some pharmaceutical benefit. Desired residual solvent may reside in either or both layers.

Coating solutions that are used in the manufacture of the multilaminated devices of the present invention are prepared and cast individually onto a support. Typically the support is composed of a poly-coated fiber paper that has enough to withstand additional coating layers a heat during the coating drying processes. While it is desirable for all components in each coating solution to be in solution, coating solutions in which one or more components are partially or fully suspended in the coating solution may also be used. Each solution is cast and processed into a thin film by techniques known in the art, such as by film dipping, film coating, film casting, spin coating, or spray drying. The thin film is then dried. This drying process may be accomplished using any suitable type of oven. However, the solvent residuals are dependent upon the drying procedure and parameters used. Alternatively, the individual layers of the multi-layered device can be produced independently and then laminated together or may be filmed one on top of the other. In one embodiment of the present invention, an adhesive layer containing a pharmaceutical is cast on a carrier support paper and dried in an oven until a continuous film is formed. Next, a water soluble polymer solution is cast on top of this adhesive layer and dried in an oven, forming a distinct laminated layer. Next, a coating solution comprising both water soluble and water insoluble polymers is cast on top film and the resulting composite film dried forming a uniform laminated device. After appropriate drying, the multilayered film obtained may be cut into any size or shape, for application to the mucosal tissue. Some possible shapes include circles, ellipses, squares, rectangles, and parallelepipeds.

Methods for producing mucoadhesive devices and treating mucosal surfaces, specifically oral mucosa, surrounding tissues, and bodily fluids for localized and systemic drug delivery are provided. In one embodiment, the method comprises applying a multi-layered, adherent film to the treatment site in order to provide drug delivery and protection to the underlying site. The adherent film may comprise any of the layered devices provided herein. In a preferred embodiment, the method comprises application of a multi-layered pharmaceutical carrier device having a first mucoadhesive layer, a water soluble backing layer that is coated to render it water erodible which regulates the residence time and erosion rate of the device. The pharmaceutical or other active compound may be present in the adhesive layer, backing layer, or both layers.

### Summary of Examples

1. Placebo adhesive for tooth surfaces
2. Backing Solution, 2:1
3. Backing Solution, 1:1
4. Backing Solution, 3:1
5. Laminating Solution to bind HPMC film to backing layers
6. Coating the precast HPMC film with various backing layers
7. Composite film to use on the surfaces of teeth
8. Mucoadhesion suspension containing Granisetron
9. Backing Solution, 3;1
10. Composite device containing Granisetron
11. Mucoadhesive suspension containing Amlexanox
12. Mucoadhesive suspension containing Benzocaine
13. Backing Solution, 2:1
14. Laminating(binding) solution
15. Composite three layer device containing Benzocaine
16. Composite three layer device containing Benzocaine
17. Composite three layer device containing Benzocaine made in reverse order
18. Mucoadhesive suspension containing Benzocaine
19. Backing Solution, 1:1
20. Backing Solution, 2:1
21. Backing Solution, 3:1
22. Coating of precast HPMC films
23. Laminating solution
24. Composite 4 layer device containing Amlexanox
25. Composite 4 layer device containing Benzocaine
26.ln vivo testing of Benzocaine Discs
27. Backing Solution, 1:1
28. Mucoadhesive suspension containing benazepril
29.Composite device containing Benazepril, 1;1
30. Backing Solution, 3: 1
31. Mucoadhesive suspension containing Benazepril
32. Composite device containing Benazepril, 3:1
33.ln Vivo testing in dogs using Benazepril discs
34. Mucoadhesive suspension containing Amlexanox
35. Binding solution
36. Backing Solution, 2:1
37. Composite 3 layer device containing Amlexanox
38. Backing Solution, 1.5:1
39. Mucoadhesive solution containing Sodium Fluoride
40. Composite strip device containing Sodium Fluoride
41. Backing Solution, 1.5:1
42. Mucoadhesive suspension containing Calcium Chloride
43. Mucoadhesive suspension containing potassium phosphate and sodium fluoride
44. Composite strip device for tooth reminerilization
45. Mucoadhesive suspension for breath freshener
46. Backing Solution, 5:1
47. Coating Solution containing peppermint oil
48. Composite long-acting breath freshener device

### Example 1

A 180.0 gram batch of placebo adhesive solution was prepared using 10.0 grams hydroxyethyl cellulose (Natrosol 250L NF; Hercules), 10.0 grams polyvinyl pyrrolidone (PVP; Povidone P-1416; Spectrum), 0.65 grams sodium benzoate (Spectrum), 0.65 grams propylene glycol (Spectrum), and 158.7 grams deionized and 0.22.mu.-fiItered water. This solution was used in Example 11 below.

### Example 2

A 19.0 gm batch of backing solution was prepared using 2.0 grams of ethyl cellulose (Ethocel Premium Std 7; Dow Chemical), 1.0 grams of HPMC (Methocel E5 Prem LV; Dow Chemical), 1.0 gram Adams Extract Red Food Color, and 15.0 grams ethanol (190 proof; USP; Spectrum). This backing solution was used in Examples 11, 15, and 18 below.

### Example 3

A 16.5 gram batch of backing solution was prepared using 1.1 grams of ethyl cellulose, 1.1 grams of HPMC, 0.9 grams Adams Extract Red Food Color, and 13.4 grams ethanol (190 proof, USP; Spectrum). This backing solution was used to make Examples 16 and 17 below.

### Example 4

A 19.8 gram batch of backing solution was prepared using 3.3 grams of ethyl cellulose, 1.1 grams of HPMC, 0.9 grams Adams Extract Red Food Color and 14.5 grams ethanol (190 proof, USP; Spectrum). This backing solution was used to make Examples 12, 13, 19, and 20 below.

### Example 5

A 52.7 gram batch of laminating solution was prepared using 6.32 grams PVP (P1416; Spectrum), 23.19 grams ethanol (190 proof, USP; Spectrum), and 23.19 grams deionized and 0.22.mu. filtered water.

### Example 6

The precast HPMC film used in several embodiments of this device was typically a 100mu. thick sheet called EM11 00 from Polymer Films. In some embodiments, it was stretched on a paper and-foil frame of a Wemer Mathis AG Lab Coater, type LTF, and a backing solution selected from Examples 2-4 was poured on top and doctor-bladed at a 0.25 mm setting, then dried in the oven section of the Lab Coater.

### Example 7

A composite device was made by doctor-blading the adhesive solution of Example 1 into a film using the Lab Coater. The casting was performed on a polymer-coated paper from Fortifiber, which was put on the paper and foil frame of the Lab Coater, with a doctor blade setting of 1.76 mm. The film was automatically dried in the oven portion of the Lab Coater, and a smooth, integral layer of deposited, adhesive polymer resulted. Then, separately, a layer of backing solution from Example 3 was doctor-bladed on top of the precast HPMC film (Example 6) using a 0.25 mm setting. The two films were then laminated together using the laminating solution described in Example 5 and pressure from a roller, followed by drying in the Lab Coater oven. The film was cut either before or after removal from the coated paper and upon application to a moist tooth surface, the film stuck well and lasted for about 15 minutes before complete erosion.

### Example 8

A 256 gram batch of active mucoadhesive suspension was prepared in two parts. One part was a mixture of 6.50 grams polyethylene glycol (Carbowax Sentry PEG-600, Dow Chemical), 1.57 grams propylene glycol (PR130, Spectrum), and 0.33 grams methylparaben (ME163, Spectrum) which was heated to about 50°C until it melted into a clear solution.

The second part was mixed in a planetary mixer (KitchenAid with a 4.5 quart bowl). It comprised 171.45 grams of water (MiIIi-Q, Millipore), 0.17 grams EDTA (ED150, Spectrum), 0.49 grams acesulfame K (A2815, Spectrum), 0.98 grams titanium dioxide (TI140, Spectrum),13.02 grams hydroxyethylcellulose (Natrosol 250L NF, Hercules), 4.87 grams polyacrylic acid (Carbopol Ultrez10 NF, Noveon), and 6.52 grams copovidone USP (Plasdone S-360, ISP Industries). The first part was added to the second part and mixed until homogeneous. Then, 4.66 grams of tragacanth (TR105, Spectrum), 5.52 grams polyvinylpyrrolidone (Kollidon K-90, BASF), 6.52 grams sodium carboxymethylcellulose (Cellulose Gum 7LF PH, Hercules), and 3.26 grams Granisetron hydrochloride (Granisetron, ChemAgis) were added. The pH was adjusted with 30.27 grams of 5% sodium hydroxide (S0170, Spectrum) at a final pH=6.25.

### Example 9

A 57.5 gram batch of backing solution composed of 3:1 hydrophobic polymer:hydrophilic polymer was prepared. Into a 250 ml plastic jar, 0.04 grams FD&C red dye #40 alum lake (Cat. 09310, Sensient) and 0.36 grams propylene glycol (PR130, Spectrum) were placed and vortex mixed (with Vortex-Genie 2, Scientific Industries). A magnetic stir bar was then added. Then 48.90 grams 190 proof ethanol (ET-108, Spectrum), 5.46 grams ethylcellulose (Ethocel Std. 7, Dow Chemical), 1.82 grams Hydroxypropylmethylcellulose (Methocel E5 LV, Dow Chemical), and 0.91 grams Triacetin (TR106, Spectrum) were added and the solution mixed until dissolved.

### Example 10

A composite device was made by doctor-blading the active mucoadhesive suspension from example 8 into a film using a Lab Coater (LTF, Wemer Mathis). The casting was performed on a polymer-coated paper from Fortifibre, which was put onto the paper and foil frame of the Lab Coater with a doctor blade setting of 1.80 mm. The film was dried in the oven portion of the Lab Coater at 50°C for 35 minutes. A smooth, integral layer of deposited adhesive polymer film resulted. A thin layer of backing layer solution from example 9 was then doctor-bladed onto the top of the dry adhesive layer using a setting of 0.65 mm. This final layer was dried in the Lab Coater oven at 50°C for 15 minutes. This bi-layer film was cut into Y2" discs using a die punch.

### Example 11

A 100 gram batch of active mucoadhesive suspension was prepared using 2.46 grams hydroxyethylcellulose (Natrosol 250LNF, Hercules), 1.25 grams Noveon AA-1 (Noveon), 0.75 grams tragacanth NF (T-300, Importers Service Corporation), 5.10 grams carboxymethylcellulose sodium (7LF PH, Hercules), 0.50 grams propylene glycol USP (PR130, Spectrum), 0.32 grams sodium benzoate NF (S0120, Spectrum), 0.05 grams edetate disodium USP (ED150, Spectrum), 0.20 grams titanium dioxide USP (T1140, Spectrum), 1.30 grams Amlexanox (Takeda), and 88.07 grams deionized and 0.22.mu.-filtered water. This suspension was used in Example 24 below.

### Example 12

A 600 gram batch of active mucoadhesive suspension was prepared in two parts. One part was a mixture of 5.04 grams propylene glycol (PR130, Spectrum) and 0.54 grams methylparaben (ME163, Spectrum) which was heated to about 50°C until it melted into a clear solution. The other part was mixed in a planetary mixer (KitchenAid with a 4.5 quart bowl). It comprised 470.70 grams of pure water (Milli-Q, Millipore), 0.24 grams EDTA (ED150, Spectrum), 1.50 grams titanium dioxide (TI140, Spectrum), 21.12 grams Hydroxyethylcellulose (Natrosol250L NF, Hercules), 1.86 grams polycarbophil (Noveon AA1, Noveon, Inc), 8.10 grams tragacanth (TR105, Spectrum), and 9.12 grams sodium alginate (S1118, Spectrum). Then the clear solution of the first part was transferred to the second part. The remaining ingredients were then added: 8.40 grams polyvinylpyrrolidone (Kollidon K-90, BASF), 8.82 grams sodium carboxymethylcellulose (Cellulose Gum 7LF PH, Hercules), and 57.00 grams benzocaine USP (micronized, Synthesia). This mixture was homogeneously blended and the pH was adjusted with 7.56 grams 10% sodium hydroxide (S0170, Spectrum) to pH 6.0.

### Example 13

A 60 gram batch of backing solution was prepared in two parts. One part was a mixture of 1.85 grams FD&C red dye #40 alum lake (Cat. 09310, Sensient) and

18.15 grams propylene glycol (PR130, Spectrum). This was blended using a vortex mixer (Vortex-Genie 2, Scientific Industries). The second part was mixed in a 250 mL jar with a magnetic stir bar. It comprised 51.75 grams 190 proof ethanol (ET-108, Spectrum), 5.20 grams ethylcellulose (Ethocel Std. 7, Dow Chemical), and 2.60 grams Hydroxypropylmethylcellulose (Methocel E5 LV, Dow Chemical). Then 0.45 grams of the first part was blended into the second part and thoroughly mixed.

### Example 14

A 350 gram batch of binding layer solution was prepared in two parts. One part was a mixture of 80.00 grams 190 proof ethanol (ET-108, Spectrum) and 20.00 grams ethyl cellulose (Ethocel Std. 7, Dow Chemical) The second part was mixed with a propeller-mixer (Lightnin G2U05R, Leeson Electric Corp.). It comprised 124.50 grams 190 proof ethanol (ET-108, Spectrum), 0.35 grams methylparaben (NE163, Spectrum), 126.00 grams pure water (Milli-Q, Millipore), and 65.8 grams Hydroxypropylmethylcellulose (Methocel E5 LV, Dow Chemical). Then 21.00 grams of the first part was blended into the second part and thoroughly mixed. Finally, 12.35 grams Triacetin (TR106, Spectrum) was added and the material was thoroughly.

### Example 15

A composite three layer device was made by doctor-blading the active mucoadhesive suspension from example 12 into a film using a Lab Coater (LTF, Werner Mathis). The casting was performed on a polymer-coated paper from Fortifibre, which was put onto the paper and foil frame of the Lab Coater with a doctor blade setting of 1.40 mm. The film was dried in the oven portion of the Lab Coater at 70°C for 25 minutes. A smooth, integral layer of deposited adhesive polymer film resulted. A thin layer of backing layer solution from example 13 was doctor-bladed onto the top of the dry adhesive layer with a doctor blade setting of 0.55 mm. This layer was dried in the Lab Coater oven at 50°C for 15 minutes. The third and final layer was made by doctor-blading the binding layer solution from example 14 onto the top of the dry 2-layer film with a doctor blade setting of 0.86 mm. This layer was dried in the Lab Coater oven at 50°C for 15 minutes. This dry, 3-layer film was die cut into 9/16" discs and the discs were stored in aluminum pouches.

### Example 16

A composite device was made by doctor-blading the active mucoadhesive suspension from example 12 into a film using a Lab Coater (LTF, Werner Mathis). The casting was performed on a polymer-coated paper from Fortifibre, which was put onto the paper and foil frame of the Lab Coater with a doctor blade setting of 1.40 mm. The film was dried in the oven portion of the Lab Coater at 70°C for 25 minutes. A smooth, integral layer of deposited adhesive polymer film resulted. The binding layer was made by doctor-blading the binding layer solution from example 14 onto the top of the dry adhesive film with a doctor blade setting of 0.85 mm. This layer was dried in the Lab Coater oven at 50°C for 15 minutes. A thin layer of backing layer solution from example 13 was doctor-bladed onto the top of the dry adhesive layer with a doctor blade setting of 0.55 mm. This final layer was dried in the Lab Coater oven at 50°C for 15 minutes. This produces a 3-layer film that could be die cut into discs.

### Example 17

The 3-layer film of Example 16 can be made by casting the 3 layers in reverse order. A composite device was made by doctor-blading the backing layer solution from example 13 into a film using a Lab Coater (LTF, Werner Mathis). The casting was performed on a polymer-coated paper from Fortifibre, which was put onto the paper and foil frame of the Lab Coater with a doctor blade setting of 0.20 mm. The film was dried in the oven portion of the Lab Coater at 50°C for 15 minutes. The binding layer was made by doctor-blading the binding layer solution from example 14 onto the top of the dry backing layer film with a doctor blade setting of 0.85 mm. This layer was dried in the Lab Coater oven at 50°C for 12 minutes. The adhesive layer was made by doctor-blading the active mucoadhesive suspension from example 12 onto the top of the dry binding layer with a doctor blade setting of 1.45 mm. This final layer was dried in the Lab Coater oven at 50°C for 20 minutes. This produces a 3-layer film that could be die cut into discs.

### Example 18

A 110.0 gram batch of active mucoadhesive suspension was prepared using 4.09 grams hydroxyethylcellulose (Natrosol 250LNF, Hercules), 1.84 grams sodium alginate NF (HF120RBS, FMC BioPolymer), 0.50 grams Noveon AA-1 (Noveon), 1.63 grams tragacanth (T-300, Importers Service Corporation), 1.69 grams povidone K90 USP (P1416, Spectrum), 1.65 grams carboxymethylcellulose sodium (7LF PH, Hercules), 1.02 grams propylene glycol USP (PR130, Spectrum), 0.10 grams methylparaben USP/NF (ME163, Spectrum), 0.05 grams edetate disodium USP (ED150, Spectrum), 0.20 grams titanium dioxide USP (T1140, Spectrum), 11.64 grams benzocaine USP (micronized, Synthesia), and 85.59 grams deionized and 0.22.mu.-filtered water. This suspension was used in Example 25.

### Example 19

A 73.35 gram batch of backing solution was prepared using 5.20 grams of ethyl cellulose (Ethocel Premium Std 7, Dow Chemical), 5.20 grams HPMC (Methocel E5 Prem LV, Dow Chemical), 0.50 grams propylene glycol USP (G1016, Spectrum), 0.05 grams of FD&C Red #40 ALUM Lake (Sensient Technologies Corporation), and 62.4 grams alcohol 190 proof USP (ET108, Spectrum). This polymer solution is denoted as a 1:1 coating solution.

### Example 20

A 73.35 gram batch of backing solution was prepared using 10.4 grams of ethyl cellulose (Ethocel Premium Std 7, Dow Chemical), 5.20 grams HPMC (Methocel E5 Prem LV, Dow Chemical), 0.50 grams propylene glycol USP (G1016, Spectrum), 0.05 grams of FD&C Red #40 ALUM Lake (Sensient Technologies Corporation), and 57.2 grams alcohol 190 proof USP (ET108, Spectrum). This polymer solution is denoted as a 2:1 coating solution.

### Example 21

A 73.35 gram batch of backing solution was prepared using 15.6 grams of ethyl cellulose (Ethocel Premium Std 7, Dow Chemical), 5.20 grams HPMC (Methocel E5 Prem LV, Dow Chemical), 0.50 grams propylene glycol USP (G1016, Spectrum), 0.05 grams of FD&C Red #40 ALUM Lake (Sensient Technologies Corporation), and 52.0 grams alcohol 190 proof USP (ET108, Spectrum). This polymer solution is denoted as a 3: 1 coating solution.

### Example 22

The precast HPMC film used to produce a variety of mucoadhesive multi-layered devices was typically a 100 microns thick film called EM 1100 from Polymer Films. The HPMC film was stretched on a paper-and-foil frame of a Werner Mathis AG Lab Coater, type LTF, and backing solutions from Examples 19, 20 and 21 were poured on top and doctor-bladed at the setting of 0.25 mm, then dried in the oven section of the Lab Coater. These backing layer coated films were used to make composite films as shown in examples 24 and 25.

### Example 23

A 100 gram batch of laminating solution was prepared dissolving 12 grams povidone K90 USP (P1416, Spectrum) in 44 grams deionized and 0.22.mu.filtered water and 44 grams alcohol 190 proof USP (ET108, Spectrum). This laminating solution was used to produce multilayered films of different residence times in Examples 24 and 25 below.

### Example 24

A composite device was made by doctor-blading the active mucoadhesive suspension of Example 11 into a film using the Lab Coater. The casting was performed on a polymer-coated paper from Fortifiber, which was put on the paper and foil frame of the Lab Coater, with a doctor blade setting of 1.90 mm. The film was automatically dried in the oven portion of the Lab Coater, and a smooth, integral layer of deposited adhesive polymer film resulted. A thin layer of the laminating solution from Example 23 was then applied to the adhesive film; and the adhesive film was laminated to the HPMC/backing layer coating composite film from Example 22 with backing layer coating facing up. Finally, the laminated film was automatically dried in the oven portion of the lab coater. This multilayered film was die cut into 1/2 inch discs and the discs were stored in heat-sealed aluminum pouches.

### Example 25

The experimental process outlined in Example 24 was repeated exactly to make another multilayer film, with the exception that the active adhesive suspension was from Example 18 and the doctor blade setting was at 1.70 mm. Two composite films were produced, one using the precast film that used the coating solution from example 20 and another using the coating solution from example 21. These multilayered films were die cut into 9/16 inch discs and the discs were stored in heat-sealed aluminum pouches.

### Example 26: In Vivo Testing of Discs from Example 25

Three human volunteers were chosen to participate in this study. Each person received five discs of both formulations. Each formulation was identical with exception that the outer coating layer varied from 2:1 hydrophobic:hydrophilic polymer composition to 3:1. The discs were assayed for benzocaine content and the average found to be 15.38 milligrams/disc. A disc from one formulation was placed on the inner cheek and after a specific time interval, the disc was carefully removed from the mucosa using a rigid spatula, ensuring that the remaining noneroded mass was completely transferred to a weigh boat and the amount of benzocaine determined analytically. Then, a second disc was applied and left for a longer time period, benzocaine analyzed, and the same process continued until 4 to 5 time points were generated. This study was repeated with discs from the second formulation and the cumulative data is shown as follows:

| | | **SUBJECT 1** | | | **SUBJECT 2** | | | **SUBJECT 3** | | |
|---|---|---|---|---|---|---|---|---|---|---|
| **Sample** | **Ethocel: Methocel Ratio** | **Time Interval (mins)** | **Retained Benzocaine (mg)** | **Benzocaine Released (mg)** | **Time Interval (mins)** | **Retained Benzocaine (mg)** | **Benzocaine Released (mg)** | **Time Interval (mins)** | **Retained Benzocaine (mg)** | **Benzocaine Released (mg)** |
| | | 15 | 12.6 | 2.73 | 16 | 13.3 | 2.03 | 20 | 12.6 | 2.68 |
| | | 25 | 11.2 | 4.14 | 30 | 11.4 | 3.92 | 30 | 11.7 | 3.66 |
| 5006 A | 2:1 | 35 | 9.14 | 6.18 | 45 | 7.93 | 7.39 | 40 | 9.68 | 5.64 |
| | | 45 | 6.67 | 8.65 | 60 | 4.93 | 10.4 | 50 | 7.88 | 7.44 |
| | | 65 | 3.7 | 11.6 | \\\\ | | | 69 | 3.13 | 12.2 |
| | | 15 | 13.9 | 1.42 | 15 | 13 | 2.33 | 15 | 13.8 | 1.53 |
| | | 30 | 11.6 | 3.71 | 30 | 12.3 | 3.02 | 30 | 12.7 | 2.61 |
| | | 45 | 10.3 | 5.04 | 30 | 11.8 | 3.52 | 45 | 11.3 | 3.99 |
| 5007 A | 3:1 | 60 | 9.89 | 5.43 | 40 | 10.8 | 4.51 | 60 | 10.1 | 5.24 |
| | | 82 | 7.38 | 7.94 | 45 | 10.2 | 5.11 | 75 | 9.19 | 6.13 |
| | | | | | 60 | 8.87 | 6.45 | | | |
| | | | | | 70 | 7.2 | 8.12 | | | |

| **Subject** | **Benzocaine Release Rate (mg/minute)** | |
|---|---|---|
| | **2:1 Backing Layer** | **3:1 Backing Layer** |
| 1 | 0.18 | 0.09 |
| 2 | 0.19 | 0.10 |
| 3 | 0.20 | 0.08 |
| **Average** | **0.19 mg/min** | **0.09 mg/min** |

### Example 27

A 76.3 gram batch of backing solution was prepared. Into a 250 mL plastic jar, 0.004 grams FD&C blue dye #1 alum lake (Cat. 09903, Sensient) and 0.05 grams propylene glycol (PR130, Spectrum) were placed and vortex mixed (with Vortex-Genie 2, Scientific Industries). A magnetic stir bar was added. Then 64.50 grams 190 proof ethanol (ET-108, Spectrum), 5.25 grams ethylcellulose (Ethocel Std. 7, Dow Chemical), 3.68 grams Hydroxypropylmethylcellulose (Methocel E5 LV, Dow Chemical), 1.58 grams polyvinylpyrrolidone (Kollidon K90, BASF), and 1.24 grams Triacetin (TR106, Spectrum) were added and the solution was mixed until dissolved.

### Example 28

A 250.0 gram batch of active mucoadhesive suspension was prepared in two parts. One part was a mix of 6.85 grams polyethylene glycol (Carbowax Sentry PEG-600, Dow Chemical), 1.73 grams propylene glycol (PR130, Spectrum), and 0.35 grams methylparaben (ME163, Spectrum) which was heated to about 50°C until it melted into a clear solution.

The second part was mixed in a planetary mixer (KitchenAid with a 4.5 quart bowl). It comprised 148.2 grams of water (Milli-Q, Millipore), 0.18 grams EDTA (ED150, Spectrum), 0.53 grams acesulfame K (A2815, Spectrum), 1.85 grams titanium dioxide (T1140, Spectrum),13.70 grams hydroxyethylcellulose (Natrosol 250L NF, Hercules), 5.13 grams polyacrylic acid (Carbopol Ultrez10 NF, Noveon), and 6.88 grams copovidone USP (Plasdone S-360, ISP Industries). Then the first part was added to this material and the total thoroughly mixed. 4.90 grams tragacanth (TR105, Spectrum), 5.80 grams polyvinylpyrrolidone (Kollidon K-90, BASF), 6.88 grams sodium carboxymethylcellulose (Cellulose Gum 7LF PH, Hercules), and 10.78 grams Benazepril Powder (Pure Benazepril Substance #100231, Novartis) were added. The pH was adjusted with 36.25 grams 10% sodium hydroxide (S0170, Spectrum) to a final pH of 6.25.

### Example 29

A composite device was made by doctor-blading a thin layer of backing layer solution from example 27 into a film using a Lab Coater (LTF, Werner Mathis). The casting was performed on a polymer-coated paper from Fortifibre, which was put onto the paper and foil frame of the Lab Coater with a doctor blade setting of 0.30 mm. The film was dried in the oven portion of the Lab Coater at 50°C for 15 minutes. A smooth, thin film resulted. The active mucoadhesive suspension from example 28 was doctor-bladed onto the top of the dry backing layer with a doctor blade setting of 0.93 mm. This final layer was dried in the Lab Coater oven at 70°C for 30 minutes. This produced a 2-layer film that was die cut into 9/16" discs and stored in a poly-lined aluminum pouch. The discs were assayed and determined to have approximately 4.9 milligrams of benazepril per disc.

### Example 30

A 76.8 gram batch of backing solution was prepared. Into a 250 mL plastic jar, 0.05 grams FD&C red dye #40 alum lake (Cat. 09310, Sensient) and 0.50 grams propylene glycol (PR130, Spectrum were placed and vortex mixed (with VortexGenie 2, Scientific Industries). A magnetic stir bar was added. Then 64.50 grams 190 proof ethanol (ET-108, Spectrum), 7.88 grams ethylcellulose (Ethocel Std. 7, Dow Chemical), 1.84 grams Hydroxypropylmethylcellulose (Methocel E5 LV, Dow Chemical) were added and

### Example 31

A 250.0 gram batch of active mucoadhesive suspension was prepared in two parts. One part was a mix of 6.85 grams polyethylene glycol (Carbowax Sentry PEG-600, Dow Chemical), 1.73 grams propylene glycol (PR130, Spectrum), and 0.35 grams methylparaben (ME163, Spectrum) which was heated to about 50°C until it melted into a clear solution.

The second part was mixed in a planetary mixer (KitchenAid with a 4.5 quart bowl). It comprised 148.2 grams of water (Milli-Q, Millipore), 0.18 grams EDTA (ED150, Spectrum), 0.53 grams acesulfame K (A2815, Spectrum), 1.85 grams titanium dioxide (TI140, Spectrum),13.70 grams hydroxyethylcellulose (Natrosol 250L NF, Hercules), 5.13 grams polyacrylic acid (Carbopol Ultrez10 NF, Noveon), and 6.88 grams copovidone USP (Plasdone S-360, ISP Industries).

Then the first part was added to this second part and the total thoroughly mixed. Then 4.90 grams tragacanth (TR105, Spectrum), 5.80 grams polyvinylpyrrolidone (Kollidon K-90, BASF), 6.88 grams sodium carboxymethylcellulose (Cellulose Gum 7LF PH, Hercules), and 10.78 grams Benazepril Powder (Pure Benazepril Substance #100231, Novartis) were added. The pH was adjusted with 36.25 grams 10% sodium hydroxide (S0170, Spectrum) to a final pH of 6.25.

### Example 32

A composite device was made by doctor-blading a thin layer of backing layer solution from example 30 into a film using a Lab Coater (LTF, Werner Mathis). The casting was performed on a polymer-coated paper from Fortifibre, which was put onto the paper and foil frame of the Lab Coater with a doctor blade setting of 0.30 mm. The film was dried in the oven portion of the Lab Coater at 50°C for 15 minutes. A smooth, thin film resulted. The active mucoadhesive suspension from example 31 was doctor-bladed onto the top of the dry backing layer with a doctor blade setting of 0.93 mm. This final layer was dried in the Lab Coater oven at 70°C for 30 minutes. This produced a 2-layer film that was die cut into 9/16" discs and stored in poly-lined aluminum pouches. The discs were assayed and determined to have approximately 4.9 milligrams of benazepril per disc.

### Example 33: In Vivo Animal Testing of Discs Produced in Examples 29 and 32.

Disc were sent to an outside Pharmaceutical Company and samples of both formulations, namely discs that had a 3: 1 outer backing layer and discs that had a 1:1 outer backing layer were attached to the oral mucosa in dogs. As a control, Lactose tablets normalized to a dose of 1 mg/kg Benazepril were administered and PK parameters for Benazeprilat, the metabolite of Benazepril, were determined. The following table summarizes the results:

| Parameter (unit) | 3:1 | 1:1 | Lactose Tablet |
|---|---|---|---|
| Tₘₐₓ^{(h)} | 2.0 | 1.7 | 1.0 |
| Cₘₐₓ (ng.h.mL⁻¹) | 214 | 201 | 121 |
| AUC_{0-inf}(ng.h.mL⁻¹) | 1200 | 827 | 511 |

These results clearly demonstrate that by controlling the backing coating layer of the mucoadhesive device, that is, with respect to its hydrophobic and hydrophilic character, the resulting blood levels of an active can be modulated. The more hydrophobic backing layer discs, 3:1, provided a higher amount of active concentration in blood that the 1:1 discs and the time to reach maximum concentration was prolonged. This clearly is a relationship between the erosion rate of the device, as the 3:1 device erodes slower and at a constant rate and stays in contact with the oral mucosa for a longer period of time than the 1:1 device. More compelling are the AUC values, as the area under the curve which show almost a 50% increase in active blood level concentration for discs having a 3:1 backing coating layer with all other parameters of the disc kept constant. By keeping the disc attached to the mucosa for a longer period of time; i.e. Slower erosion rate, the drug has a higher probability to enter the intercellular pathway and provide a higher drug level in blood.

### Example 34

A 300.0 gram batch of active mucoadhesive suspension was prepared in a planetary mixer (KitchenAid with a 4.5 quart bowl). This batch was composed of 266.51 grams water (Milli-O, Millipore), 5.34 grams Hydroxyethylcellulose (Natrosol 250L NF, Hercules), 18.04 grams sodium carboxymethylcellulose (Cellulose Gum 7LF PH, Hercules), 0.66 grams titanium dioxide (T1140, Spectrum), 0.16 grams EDTA (ED150, Spectrum), 4.35 grams Amlexanox (sieved to less than 150Il, Takeda), 0.96 grams sodium benzoate (S0120, Spectrum), 0.96 grams propylene glycol (PR130, Spectrum), 3.02 grams polycarbophil (Noveon AA1, Noveon, Inc). The mixture was blended for at least 2 hours until homogeneous.

### Example 35

A 150.0 gram batch of binding layer solution was prepared in a 250 mL jar with a propeller-mixer (Lightnin G2U05R, Leeson Electric Corp.). It comprised 105.00 grams pure water (MiIIi-Q, Millipore), 28.13 grams Hydroxypropylmethylcellulose (Methocel E5 LV, Dow Chemical), and 16.87 grams polyvinylpyrrolidone (Kollidon K-90, BASF).

### Example 36

A 50.0 gram batch of backing solution was prepared. 0.48 grams FD&C red dye #40 alum lake (Cat. 09310, Sensient) and 1.27 grams propylene glycol (PR130, Spectrum) were placed into a plastic 250 mL jar and vortex mixed (with VortexGenie 2, Scientific Industries). A magnetic stir bar was added. Then 40.30 grams 190 proof ethanol (ET-108, Spectrum), 5.30 grams ethylcellulose (Ethocel Std. 7, Dow Chemical), 2.65 grams Hydroxypropylmethylcellulose (Methocel E5 LV, Dow Chemical) were added and the mixture blended until homogeneous.

### Example 37

A composite three layer device containing Amlexanox was produced by doctorblading the active mucoadhesive suspension from example 34 into a film using a Lab Coater (LTF, Werner Mathis). The casting was performed on a polymercoated paper from Fortifibre, which was put onto the paper and foil frame of the Lab Coater with a doctor blade setting of 1.59 mm. The film was dried in the oven portion of the Lab Coater at 70°C for 13 minutes. A smooth, integral layer of deposited adhesive polymer film resulted. The binding layer was made by doctor-blading the binding layer solution from example 35 onto the top of the dry adhesive film with a doctor blade· setting of 0.85 mm. This layer was dried in the Lab Coater oven at 50°C for 20 minutes. A thin layer of backing layer solution from example 36 was doctor-bladed onto the top of the dry 2-layer film with a doctor blade setting of 0.47 mm. This final layer was dried in the Lab Coater oven at 50°C for 15 minutes. This produced a 3-layer film that was die cut into ½" discs.

### Example 38

A 73.0 gram batch of backing solution was prepared. Put 0.05 grams FD&C red dye #40 alum lake (Cat. 09310, Sensient) and 0.46 grams propylene glycol (PR130, Spectrum) into a plastic 250 mL jar and vortex mix (using Vortex-Genie 2, Scientific Industries). A magnetic stir bar was added. Then 63.01 grams 190 proof ethanol (ET-108, Spectrum), 5.69 grams ethylcellulose (Ethocel Std. 7, Dow Chemical), and 3.79 grams Hydroxypropylmethylcellulose (Methocel E5 LV, Dow Chemical) were added and the material homogenized.

### Example 39

A 150.0 gram batch of active mucoadhesive solution was prepared in two parts. One part was a mixture of 4.38 grams propylene glycol (PR130, Spectrum) and 0.22 grams methylparaben (ME163, Spectrum) which was heated to about 50°C until it melted into a clear solution.

The second part was mixed in a planetary mixer (KitchenAid). It comprised 95.88 grams of pure water (Milli-Q, Millipore), 0.44 grams acesulfame K (A2815, Spectrum), 0.88 grams titanium dioxide (TI140, Spectrum),24.11 grams hydroxyethylcellulose (Natrosol 250L NF, Hercules), and 24.11 grams polyvinylpyrrolidone (Kollidon K-90, BASF). Then the first part was mixed into this second part and the mixture was thoroughly blended. Finally, 0.19 grams sodium fluoride (S0167, Spectrum) were added and mixed for an additional 30 minutes.

### Example 40

A composite device was made by doctor-blading a thin layer of backing layer solution from example 38 into a film using a Lab Coater (LTF, Werner Mathis). The casting was performed on a polymer-coated paper from Fortifibre, which was put onto the paper and foil frame of the Lab Coater with a doctor blade setting of 0.32 mm. The film was dried in the oven portion of the Lab Coater at 50°C for 15 minutes. A smooth, thin film resulted. The active mucoadhesive solution from example 39 was doctor-bladed onto the top of the dry backing layer with a doctor blade setting of 0.57 mm. This final layer was dried in the Lab Coater oven at 50°C for 15 minutes. This produced a 2-layer, fluoride containing film that was die cut into 19/32"x 2 ¾" strips.

### Example 41

A 55.62 gram batch of backing solution was prepared. Into a plastic 250 mL jar, 0.003 grams FD&C blue dye #1 alum lake (Cat. 09903, Sensient) and 0.30 grams propylene glycol (PR130, Spectrum) were placed and vortex mixed (using Vortex-Genie 2, Scientific Industries). A magnetic stir bar was then added. Then, 48.40 grams 190 proof ethanol (ET-108, Spectrum), 3.72 grams ethylcellulose (Ethocel Std. 7, Dow Chemical), 2.48 grams Hydroxypropylmethylcellulose (Methocel E5 LV, Dow Chemical), and 0.72 grams Triacetin (TR106, Spectrum) were added and the mixture homogenized.

### Example 42

A 276.9 gram batch of mucoadhesive suspension containing calcium chloride was prepared in two parts. One part was a mixture of 6.56 grams propylene glycol (PR130, Spectrum) and 0.33 grams methylparaben (ME163, Spectrum) which was heated to about 50°C until it melted into a clear solution. The second part was mixed in a planetary mixer (KitchenAid). First 171.9 grams of water (Milli-Q, Millipore) and 36.07 grams polyvinylpyrrolidone (Kollidon K-90, BASF) were added. After this was homogenized, the first part was mixed into this second part and thoroughly mixed.

Then 36.07 grams hydroxyethylcellulose (Natrosol 250L NF, Hercules), 0.66 grams acesulfame K (A2815, Spectrum), and 1.31 grams titanium dioxide (TI140, Spectrum) were added.

Finally, 9.00 grams calcium chloride dihydrate (CA140, Spectrum) were dissolved in 10.0 grams of water. This solution was rinsed into the main part with another 5.0 grams of water. The batch was blended until homogeneous.

### Example 43

A 300 gram batch of mucoadhesive suspension containing potassium phosphate was prepared in two parts. One part was a mixture of 6.54 grams propylene glycol (PR130, Spectrum), 5.45 grams glycerin (G1 016, Spectrum), 10.90 grams polyethylene glycol (Carbowax Sentry PEG-600, Dow Chemical), 0.68 grams polysorbate 80 (P0138, Spectrum), and 0.36 grams methylparaben (ME163, Spectrum). This mixture was heated to about 50°C until it melted into a homogeneous solution.

The second part was mixed in a planetary mixer (KitchenAid). First 180.0 grams of water (Milli-Q, Millipore), 10.89 grams polyvinylpyrrolidone (Kollidon K-90, BASF), 38.14 grams Hydroxypropyl cellulose (Klucel EF PHARM, Hercules), and 27.24 grams copovidone USP (Plasdone S-360, ISP Technologies) were added. After this was homogeneous, the first part was mixed into this second part until homogeneous. Finally, 1.80 grams sodium fluoride (S0167, Spectrum) and 18.00 grams potassium phosphate dibasic (P0204, Spectrum) were added. The batch was thoroughly blended until homogeneous.

### Example 44

A composite device suitable for tooth enamel reminerization was made by doctor-blading a thin layer of backing layer solution from example 41 into a film using a Lab Coater (LTF, Werner Mathis). The casting was performed on a polymer-coated paper from Fortifibre, which was put onto the paper and foil frame of the Lab Coater with a doctor blade setting of 0.32 mm. The film was dried in the oven portion of the Lab Coater at 50°C for 15 minutes. A smooth, thin film resulted. The calcium chloride-mucoadhesive suspension from example 42 was doctor-bladed onto the top of the dry backing layer with a doctor blade setting of 0.74 mm. This final layer was dried in the Lab Coater oven at 50°C for 30 minutes. The potassium phosphate-fluoride--mucoadhesive suspension from example 43 was doctor-bladed onto the top of the dry 2-layer film with a doctor blade setting of 0.85 mm. This was dried in the Lab Coater at 33°C for 40 minutes and then 50°C for 70 minutes. This produced a 3-layer film that was die cut into 19/32"x 2 ¾" strips.

### Example 45

A 100.2 gram batch of adhesive solution was prepared using 4.67 grams hydroxyethyl cellulose (Natrosol 250L NF; Hercules; RM4D08), 2.61 grams PVP (Povidone P-1416; Spectrum; RM3H34), 3.00 grams propylene glycol (Spectrum), 0.45 grams titanium dioxide (PR130; Spectrum; RM1G09), 1.26 grams sodium alginate (S1118; Spectrum; RM2D44), 1.90 grams tragacanth (TR105; Spectrum; RM2K16), and 89.13 grams deionized and 0.22 g-filtered water. This solution was used in Example 82 below.

### Example 46

A 60.61 gram batch of backing solution was prepared using 10.00 grams of ethyl cellulose (Ethocel Std 7 NF; Dow Chemical; RM1 M32), 2.00 grams of HPMC (Methocel E5 PREM LV; Dow Chemical; RM1M30), 33.00 grams ethanol (190 proof, USP; Spectrum; RM4D16), 0.60 g green food dye (Kroger), and 15.01 grams of peppermint oil (USP, Spectrum; RM4E14). This solution was used in Example 82 below.

### Example 47

A 18.07 gram batch of backing solution was prepared using 1.33 grams of HPMC (Methocel E5 PREM LV; Dow Chemical; RM1M30), 4.44 grams of peppermint oil (USP, Spectrum; RM4EI4), 0.33 g red food dye (Adams Extract Red Food Coloring; RM3A01), and 11.97 grams ethanol (190 proof, USP; Spectrum; RM4DI6). This backing solution was also used in the manufacture of Example 82 below.

### Example 48

A composite, multilayered breath freshener device was made by doctor-blading the adhesive solution of Example 45 into a film using the Lab Coater. The casting was performed on a polymer-coated paper from Fortifiber, which was put on the paper and foil frame of the Lab Coater, with a doctor blade setting of 2.20 mm. The film was automatically dried in the oven portion of the Lab Coater. A smooth, integral layer of deposited, adhesive polymer resulted. Then, a layer of 5:1 hydrophobic:hydrophilic solution from Example 46 was doctor-bladed on top of the adhesive film using a 1.40 mm setting. This bilayer film was dried in the Lab Coater at 50°C. Lastly, the outer layer solution of Example 47 was spread on top of the 5:1 film. This trilayer film was dried in the Lab Coater at 50°C. The finished film could be cut either before or after removal from the coated paper. A 1/2" disc was put on the roof of the mouth. The disc stuck well and delivered an initial burst of mint flavor, and a sustained mint flavor for almost 4 hours. hose skilled in the art will recognize that, while specific embodiments and examples have been described, various modifications and changes may be made without departing from the scope and spirit of this invention.

**U.S. Patent & Application Documents**

| | | |
|---|---|---|
| 3948254 | April 1976 | Zaffaroni |
| 3993072 | November 1976 | Zaffaroni |
| 3996934 | December 1976 | Zaffaroni |
| 4226848 | October 1980 | Nagai et al. |
| 4250163 | February 1981 | Nagai et al. |
| 4286592 | September 1981 | Chandrasekaran |
| 4292299 | September 1981 | Suzuki et al. |
| 4517173 | May 1985 | Kizawa et al. |
| 4518721 | May 1985 | Dhabhar et al. |
| 4552751 | November 1985 | Inaba et al. |
| 4572832 | February 1986 | Kigasawa et al. |
| 4615697 | October 1986 | Robinson |
| 4713243 | December 1987 | Schiraldi et al. |
| 4715369 | December 1987 | Suzuki et al. |
| 4894232 | January 1990 | Reul et al. |
| 4900554 | February 1990 | Yanagibashi et al. |
| 4915948 | April 1990 | Gallopo et al. |
| 5047244 | September 1991 | Sanvordeker et al. |
| 5081157 | January 1992 | Pomerantz |
| 5081158 | January 1992 | Pomerantz |
| 5137729 | August 1992 | Kuroya et al. |
| 5139023 | August 1992 | Stanley et al. |
| 5192802 | March 1993 | Rencher |
| 5291887 | March 1994 | Stanley et al. |
| 5298258 | March 1994 | Akemi et al. |
| 5314915 | May 1994 | Rencher |
| 5458879 | October 1995 | Singh et al. |
| 5462749 | October 1995 | Rencher |
| 5474768 | December 1995 | Robinson |
| 5543150 | August 1996 | Bologna et al. |
| 5578315 | November 1996 | Chien et al. |
| 5624677 | April 1997 | El-Rashidy et al. |
| 5667492 | September 1997 | Bologna et al. |
| 5750134 | May 1998 | Scholz et al. |
| 5750136 | May 1998 | Scholz et al. |
| 5766620 | June 1998 | Heiber et al. |
| 5780045 | July 1998 | McQuinn et al. |
| 5783207 | July 1998 | Stanley et al. |
| 5800832 | September 1998 | Tapolsky et al. |
| 5827525 | October 1998 | Liao et al. |
| 5849322 | December 1998 | Ebert et al. |
| 5855908 | January 1999 | Stanley et al. |
| 5861174 | January 1999 | Stratton et al. |
| 5863555 | January 1999 | Heiber et al. |
| 5869082 | February 1999 | Dugger, III |
| 5888534 | March 1999 | EI-Rashidy et al. |
| 5900230 | May 1999 | Cutler |
| 5908637 | June 1999 | Benes et al. |
| 5955097 | September 1999 | Tapolsky et al. |
| 5955098 | September 1999 | Dugger, III |
| 5968500 | October 1999 | Robinson |
| 5981499 | November 1999 | Hau |
| 6017521 | January 2000 | Robinson et al. |
| 6071959 | June 2000 | Rhodes, et al. |
| 6103226 | August 2000 | Kang, et al. |
| 6103266 | August 2000 | Tapolsky et al. |
| 6110486 | August 2000 | Dugger, III |
| 6117446 | September 2000 | Place |
| 6159498 | December 2000 | Tapolsky et al. |
| 6166044 | December 2000 | Sandborn et al. |
| 6585997 | July 2003 | Moro, et al. |
| US2006/0073174 | April 2006 | Moro, et al. |

**Foreign Patent Documents**

| | | |
|---|---|---|
| 0765664 | Apr., 1997 | EP |
| 0765664 | Apr., 1997 | EP |
| 1593097 | Jul., 1981 | GB |
| 1593097 | Jul., 1981 | GB |
| 59186913 | Oct., 1984 | JP |
| 9609829 | Apr., 1996 | WO |
| 9609829 | Apr., 1996 | WO |
| 9801112 | Jan., 1998 | WO |
| 9801112 | Jan., 1998 | WO |
| WO 98/17251 | Apr., 1998 | WO |
| WO 99/55312 | Nov., 1999 | WO |
| 9963986 | Dec., 1999 | WO |
| WO 00/42959 | Jul., 2000 | WO |
| 0050078 | Aug., 2000 | WO |
| 02/09637 | Feb., 2002 | WO |
| 0209637 | Feb., 2002 | WO |

### Other References

Dr. Marcello Innocenti, Clinical Evaluation of Gelcair Concentrated Oral Gel, a new option for treating painful oral conditions, pp. 1-14, Milan, Italy. PDR 21 Edition 2000, Physicians Desks Reference for Nonprescription Drugs and Dietary Supplements, pp. 640 and 787..
Dr. Heddie Sedano, Oral Complications During Cancer Treatment, Jun. 13, 2002, pp. 1-5..
Jelka Korbar-Smid et aI, An Oxtetracycline Formulation to be Applied at the Oral Mucosa, Aug. 5, 1975, pp. 271-276, University of Ljubljana..
Dixon J, Search Conducted on hyaluronic Acid and Mucositis, May 29, 2002, pp. all, US Kaken Seiyaku KK, Abstract, JP 59186913, Apr. 4, 1983.. ..
Tsumura & Co, Abstract JP 8291083, Apr. 17,1995..
Jian-Hwa, et al Bioadhesive Polymer Buccal Patches for Buprenorphine Controlled Delivery: Solubility; 1995; 2013-1019; Drug Development and Industrial Pharmacy..
Hussain, et al Improved Buccal Delivery of Opioid Analgesics and Antagonist with Bitterness Prodrugs; 1998; 615-618; Pharmaceutical Research..
Badawy, et al. Bioavailability of danazol-hydroxypropyl-Beta-cyclodextrin comples by different routes of administration; 1996; 137-143; International Journal of Pharmaceutics..
Kutcher, et al Evaluation of a bioadhesive device for the management of aphthous ulcers; 1998-2001; 1-13; Advances in Dental Products..
Hoogstraate, et al Buccal delivery of fluorescein isothiocyanate-dextran 4400 and peptide drug buserelin with glycodeoxycholate as adsorption enhancer in pigs; 1996; 77-84; Journal of Controlled Release..
Li, et al Evaluation of a Mucoadhesive Buccal Patch for Delivery of Peptides; In Vitro Screening of Bioadhesion; 1998; 919-926; drug Development and Industrial Pharmacy..
Nielsen, et al Bioadhesive drug delivery systems 1. Characterisation of mucoadhesive properties of systems based on glyceryl mono-oleate and glyceryl monolinoleate; 1998; 231-239; European Journal of Pharmaceutical Sciences.. Warren The Synthesis and In Vitro Characterization of the Mucoadhesion and Swelling of Poly(Acrylic Acid) Hydrogels; 1998; 199-208; Pharmaceutical Development and Technology..
Tamburic, et al The Effects of Ageing on the Pheological, Dielectric and Mucoadhesive Properties of Poly(Acrylic Acid) Gel System; 1996; 279-283; Pharmaceutical Research..
Pather, et al Enhanced Buccal Delivery of Fentanyl Using The Oravescent.TM. Drug Delivery System; Cima Labs Inc..
Kim, et al Buccal Delivey of Butorphanol Tartrate By Using Mucoadhesive P(AA-co-PEGMM) Film; ; 361-763; College ofPharmacy, Chungbuk National University, Cheongju, Korean..
Kremer, et al Permeabilizing Effect of Different Chitosans on Drug Delivery Across Buccal Mucosa; Dows Institute For Dental Research; University of Iowa, Iowa City, Iowa..
Ali, et al In Vivo Evaluation of Bioadhesive Erodible Disk of Cetylpyrodinium Chloride and Comparison of Its Effect With Mouthwash In Oro-Dental Infections; Department of Pharmaceutics, Faculty of Pharmacy..
Kalra, et al Bilayered Buccoadhesive Wafers of An Antihypertensive Agent: Formulation and Evaluation; Department of Pharmaceutics..
Gandhi, et al Oral Mucosal Drug Delivery System of Trimetazidine Dihydrochloride; Pharmaceutical Division, University Department of Chemical Technology.

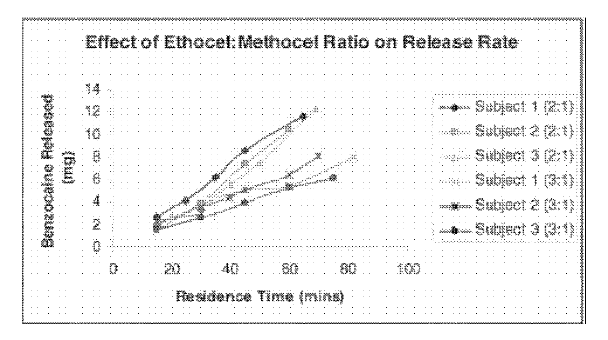

## Claims

1. A water-erodible coating layer for controlling the residence time and erosion rate of a water-soluble thin-film device that is adhered to a moist surface of a body tissue, comprising at least one hydrophobic polymer and at least one water-soluble polymer wherein the ratio of the hydrophobic polymer(s) to the water soluble polymer(s) is from about 1:1 to about 5:1 by weight.

2. A water-erodible coating layer of claim 1, wherein the layer is attached to an additionallayer(s), at least one of which is an adhesive layer and provides an erosion rate of the device independent of the radius of the device.

3. The device of claim 1, wherein the erosion rate controls the release rate of any active incorporated in the device at essentially a pseudo zero order rate.

4. The water-erodible coating layer of any preceding claim, wherein the water-soluble polymer(s) is/are selected from the group consisting of polyvinyl pyrrolidone, polyvinyl alcohol, hydroxyethyl cellulose, hydroxypropyl cellulose and hydroxypropylmethyl cellulose.

5. The water-erodible coating layer of any preceding claim, wherein the hydrophobic polymer is selected from the group consisting of methyl cellulose; ethyl cellulose; anionic, cationic and neutral methacrylate polymers and co-polymers (Eudragit.RTM. polymers); and a combination thereof.

6. The water-erodible coating layer of claim 1, further comprising a plasticizer.

7. The water-erodible coating layer of claim 6, wherein the plasticizer is selected from the group consisting of propylene glycol, polyethylene glycol and glycerin.

8. The water-erodible coating layer of claim 1, further comprising a humectant.

9. The water-erodible coating layer of claim 8, wherein the humectant is selected from the group consisting of hyaluronic acid and glycolic acid.

10. The water-erodible coating layer of claim 1, further comprising a coloring agent and/or an opacifying agent.

11. The water-erodible coating layer of claim 1, further comprising a flavor and/or a taste-masking agent.

12. A water-erodible thin-film device comprising the water-erodible coating layer of claim 1 which is coated on a water soluble polymer film layer that is formed in situ and at least one additional layer which is adhesive, wherein the layers are mechanically bonded together and capable of adhering to a moist surface of a body tissue.

13. The device of claim 12, further comprising a water soluble outer coating layer to improve mouth feel.

14. The device of claim 12 or 13,further comprising one or more agents selected from the group consisting of pharmaceutical actives, nutraceuticals, cosmeceuticals, vitamins, botanical extracts, flavors or fragrances are incorporated in one or more layers of the film.

15. The device of claims 12, 13 or 14, wherein the weight of the device is from about 0.01 gram to about 10.0 grams.
